# EUROPÄISCHE PATENTSCHRIFT

(11) **EP 2 389 961 B1**
(45) Veröffentlichungstag und Bekanntmachung des Hinweises auf die Patenterteilung: **27.03.2013**
(21) Anmeldenummer: 11004254.6
(22) Anmeldetag: 24.05.2011
(51) Int. Cl.: A61M 1/00

(54) **Medizinisches Gerät zur Verbesserung des Wundheilungsprozesses**
Medical device for improving the wound healing process
Appareil médical destiné à l'amélioration du processus de cicatrisation de plaies

(30) Priorität: 31.05.2010 DE 102010022060
(43) Veröffentlichungstag der Anmeldung: 30.11.2011
(73) Patentinhaber: ORTHOS Orthopädietechnik GmbH, 22765 Hamburg (DE)
(72) Erfinder: Schleusener, Reiner, 22559 Hamburg (DE)
(74) Vertreter: Groth, Wieland

(56) Entgegenhaltungen:
- WO-A2-2009/089390
- US-A1- 2007 055 209

## Beschreibung

Die Erfindung betrifft ein medizinisches Gerät zur Verbesserung eines Wundheilungsprozesses eines Patienten, insbesondere bei durchblutungsgestörtem Gewebe.

Medizinische Geräte sind in Form von intermittierenden Vakuumtechniken und Wundabsaugvorrichtungen im Stand der Technik bekannt.

In der DE 600 37 824 T2 ist eine Wundabsaugvorrichtung beschrieben. Dabei wird ein an einen Absaugschlauch angeschlossenes poröses Kissen auf eine Wunde aufgebracht, der mit einer Unterdruckquelle verbunden ist. Die Wundabsaugvorrichtung ermöglicht es, Wundexsudat aus der Wunde zu saugen und zu sammeln. Wundexsudate sind bei entzündlichen Prozessen des Wundödems erzeugte, vom Blutplasma abgeleitete Wundflüssigkeiten.

In der WO 96/05873 ist ein therapeutisches Gerät zur Anregung des Heilungsprozesses von Wunden offenbart. Auch hier wird ein Unterdruck über der Wunde erzeugt, um Wundflüssigkeit abzusaugen und dadurch den Heilungsprozess zu befördern.

Auch aus der DE 10 2008 034363 A1 ist ein Wundpflegeartikel mit absorbierender Hülle bekannt, die es ermöglicht, die bei länger andauernden Heilungsprozessen erzeugten Exsudate abzusaugen, weil diese aufgrund einer Konzentrationsverschiebung ihrer einzelnen Bestandteile normalerweise ihre heilungsfördernde Wirkung verlieren.

In der WO 2009/089390 A2 ist ein Unterdruckgerät offenbart, mit dem Wundexsudate abgesaugt werden können. Dort ist der Herzschlag mit dem zum Absaugen von Wundexsudat benötigten Unterdruck synchronisiert.

Nachteilig an den genannten medizinischen Geräten ist, dass diese zur Beschleunigung der Wundheilung lediglich die Wundexsudate absaugen.

Daneben sind Vakuumtechniken bekannt, bei denen Wundbereiche einem intermittierenden Unterdruck ausgesetzt werden. Nachteilig an den Vakuumtechniken ist, dass die erzeugten Druckverhältnisse in keiner Weise auf die tatsächlich herrschende Durchblutungssituation eingehen. Hier wird zwar intermittierend oder permanent Blut durch Unterdruck in den Wundbereich gefördert, aber das wirkliche Wundklima, im Sinne der natürlichen Durchblutung außer Acht gelassen.

In beiden Methoden wird zwar auf die Wunde eingewirkt, die Wunde wird aber auch in ihrer sensiblen Selbstheilungsphase gestresst.

Es ist Aufgabe der vorliegenden Erfindung, ein medizinisches Gerät zur Verfügung zu stellen, das Unterdruckimpulse über einer Wunde erzeugt.

Diese Aufgabe wird durch ein eingangs genanntes medizinisches Gerät mit den Merkmalen des Anspruchs 1 gelöst.

Die Erfindung macht von der Idee Gebrauch, zur Beschleunigung des Wundheilungsprozesses die Durchblutung des Wundbereichs gezielt so anzuregen, dass der Unterdruck in dem Moment erzeugt wird, wo das Blut in den Wundbereich durch den Pulsschlag einfließt und bei Abflachen der Pulskurve der Unterdruck wieder nachlässt. Es wird eine Wechseldruckkurve, die einen Wechseldruck auf der Wunde des Patienten bewirkt, erzeugt. Der Wechseldruck wirkt dabei vorzugsweise auf den gesamten Wundbereich, indem im gesamten Wundbereich ein gleicher Unterdruck erzeugt wird. Gerade bei durchblutungsgestörtem Gewebe ist eine externe, durchblutungsfördernde Unterstützung besonders hilfreich. Der gemessene Puls wird in den Wundheilungsprozess eingebunden. Die Wundheilung ist ein körpereigener Prozess, der sich nur anregen oder beschleunigen lässt, indem wir ein günstiges Klima schaffen, in dem es dem Körper möglich ist, selbst Gewebezellen zu bilden, um die Wunde zu schließen.

Realisieren lässt sich diese Anwendung, indem der Pulsschlag des Patienten mit in die Steuerung der Pumpe, vorzugsweise Wechseldruckpumpe, eingebunden wird. Die Pumpe arbeitet pulssynchron und fördert so die Durchblutung des durchblutungsgestörten Gewebes.

Dabei wird ein die Wunde während der Behandlung vollständig abdeckender Saugaufsatz zur Verfügung gestellt, der vorzugsweise ausschließlich mit gesunden Hautbereichen des Patienten, vorzugsweise im Wesentlichen luftdicht abschließt. Der Saugaufsatz bildet einen über dem gesamten Wundbereich gleichen Wechseldruck aus, der unabhängig vom Außendruck steuerbar ist. Erfindungsgemäß wird ein pulssynchrones Wechseldrucksignal erzeugt. Das Wechseldrucksignal ist günstigerweise mit dem ständig gemessenen Puls des Patienten synchronisiert. Das bedeutet, jeder Pulsschlag erzeugt ein Wechseldrucksignal. Der Begriff Saugaufsatz ist hier breit zu verstehen. Es kann sich dabei auch um eine Saugvorrichtung, z. B. in Tonnenform handeln, in die Körperteile des Patienten, ggf. der ganze Rumpf des Patienten, eingeschoben werden.

Das Innendrucksignal umfasst vorzugsweise mit den Pulsimpulsen synchronisierte periodische Innendruckimpulse, die als Unterdruckimpulse ausgebildet sein können.

In einer Weiterbildung der Erfindung ist das Innendrucksignal mit dem aus dem gemessenen Puls durch günstigerweise die Steuereinrichtung berechneten Puls im Wundbereich synchronisiert. Synchronisiert bedeutet hier insbesondere, dass Wechseldruckimpulse und Pulsschläge des Pulses, vorzugsweise des Pulses im Wundbereich, zeitlich zusammenfallen.

Dadurch wird vorteilhafterweise, wenn ein Pulsschlag im Wundbereich auftritt, die Wunde gleichzeitig durch den Wechseldruckimpuls stimuliert, vorzugsweise ist der Wechseldruckimpuls ein Unterdruckimpuls, so dass die Wunde mit jedem Pulsschlag gleichzeitig einem Unterdruckimpuls ausgesetzt wird. Dadurch werden die Durchblutung des Wundbereichs und der Heilungsprozess besonders befördert. Zusätzlich wird somit zwischen den Unterdruckimpulsen der Abtransport von Wundflüssigkeit auf natürliche Weise unterstützt.

Die Steuereinrichtung ist vorzugsweise von einer Absaugvorrichtung für das Wundsekret getrennt. Die Steuereinrichtung erzeugt Wechseldruck im Wundbereich zur Stimulation des Wundbereichs, während das Wundsekret vorzugsweise über einen in dem Verband gelegten Absaugschlauch kontinuierlich abgesaugt wird. Die Steuereinrichtung des Saugaufsatzes für den Wechseldrucks ist von der Absaugeinrichtung des Wundsekrets entkoppelt.

Das medizinische Gerät weist zur Messung eines Pulses des Patienten einen am Patienten angeordneten Pulsmesser auf. Da der Wundbereich durch den Saugaufsatz abgedeckt ist, wird der Pulsmesser ein Stück beabstandet vom Wundbereich am Patienten angebracht. Da der Pulsmesser vom Wundbereich beabstandet ist, ist die Laufzeit der Pulsschläge zwischen dem mit dem Pulsmesser gemessenen Puls im Messbereich und dem Puls im Wundbereich bei der Steuerung zu berücksichtigen. Die Laufzeitverzögerung wird empirisch bestimmt und in einer Laufzeittabelle festgehalten.

Die Steuereinrichtung ist vorzugsweise als elektronische Steuereinrichtung ausgebildet. Die Steuereinrichtung wertet den gemessenen Puls aus und erzeugt ein Steuersignal mit dem eine mit Saugaufsatz luftleitend in Verbindung stehende Pumpe beaufschlagt wird. Vorzugsweise besteht das Steuersignal aus periodischen Steuerimpulsen, wobei die Periodenlänge bzw. die Frequenz des Steuersignals der Frequenz des gemessenen Pulses entspricht.

Steuerimpulse des Steuersignals weisen günstigerweise jeweils eine positive und negative Halbwelle auf, die nicht notwendigerweise symmetrisch sein müssen. Das der Pumpe zugeführte Steuersignal steuert einen Antrieb der Pumpe, vorzugsweise in Form eines Servo-Motors, der vorzugsweise einen Stempel zu einer Hin- und Herbewegung antreibt. Es sind aber auch andere Pumpenvarianten denkbar.

Aufgrund jedes einzelnen Steuerimpulses führt der Stempel eine impulsartige Hin-und Herbewegung aus, die zeitlich verzögert einen Wechseldruckimpuls im Saugverband erzeugt. Der Wechseldruckimpuls erfolgt zeitlich verzögert. Vorzugsweise empirisch erfasste Verzögerungen können beispielsweise in einem Speicher der Steuereinrichtung als Laufzeittabelle abgelegt sein. Die Laufzeittabelle erfasst die aufgrund der unterschiedlichen Entfernungen zwischen verschiedenen Messbereichen des Pulses und verschiedenen Wundbereichen, der Gefäßgröße u. Ä. erzeugten verschiedenen Laufzeiten des Pulses zwischen Messbereichen und Wundbereichen. Die Steuerung kann eine Eingabe aufweisen, über die die im jeweiligen Fall bestimmten Mess- und Wundbereiche eingebbar sind.

Günstigerweise ist am Saugaufsatz ein das Wechseldrucksignal erfassender und der Steuereinrichtung zuführender Drucksensor angeordnet.

Die Steuereinrichtung vergleicht den Zeitpunkt des Auftretens der Wechseldruckimpulse mit einem berechneten Zeitpunkt des Auftretens des Pulsschlages im Wundbereich und verschiebt ggf. das periodische Steuersignal um eine so bestimmte Phase, dass die Wechseldruckimpulse mit den Pulsschlägen im Wundbereich zusammenfallen.

Die Steuereinrichtung kann neben einer Phasenverschiebung des Steuersignals auch den Flankenverlauf der Steuerimpulse des Steuersignals einstellbar verändern, so dass der Flankenverlauf sowie die zeitliche Ausdehnung der Innendruckimpulse variabel gestaltbar sind.

Das erfindungsgemäße medizinische Gerät kann auch unabhängig von einer tatsächlich vorhandenen Wunde am Patienten zur Durchblutungsförderung bestimmt sein und verwendet werden.

Die Erfindung wird anhand eines Ausführungsbeispiels an drei Figuren beschrieben. Dabei zeigen:
- Fig.1: eine schematische Ansicht des erfindungsgemäßen medizinischen Unterdruckgerätes,
- Fig. 2: eine zeitlich korrelierte Darstellung vom Puls im Messbereich, Steuersignal, Innendruck und Puls im Wundbereich,
- Fig. 3: das medizinische Unterdruckgerät in einer Anwendung.

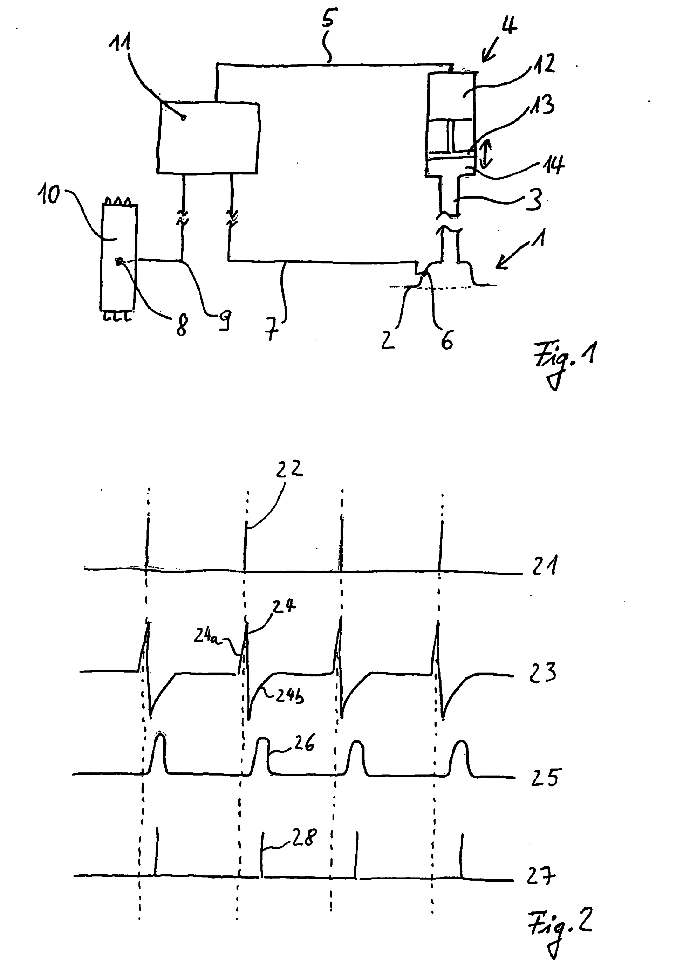

Fig. 1 zeigt die wesentlichen Bestandteile des erfindungsgemäßen medizinischen Wechseldruckgerätes in einer schematischen Funktionsansicht. Die Darstellung ist nicht maßstabsgetreu.

Ein Vakuumverband 1 ist mit einem Abschluss 2 für gesunde Hautpartien um einen Wundbereich eines Patienten versehen. Der aufgelegte Vakuumverband 1 überdeckt den Wundbereich vollständig, vorzugsweise ohne mit ihm direkt in Kontakt zu geraten.

Der Vakuumverband 1 ist zum einen über einen Luftschlauch 3 mit einer Pumpe 4 verbunden, zum anderen ist am Vakuumverband 1 ein über eine elektrische Leitung 7 mit einer Steuerung 11 verbundener Drucksensor 6 vorgesehen. Der Vakuumverband 1 wird über der zu behandelnden Wunde eines Patienten angeordnet und mit dem Abschluss 2 auf gesunden Hautpartien des Patienten mit Klebebändern möglichst luftdicht abgedichtet.

Die über den Luftschlauch 3 mit dem Vakuumverband 1 in Verbindung stehende Pumpe 4 ermöglicht es, einen vom Außendruck unabhängigen, in der Höhe, Frequenz usw. steuerbaren Wechseldruck im Vakuumverband 1 zu erzeugen. Die Pumpe 4 ist dazu über ein Steuerkabel 5 mit der Steuerung 11 elektrisch verbunden. Die Pumpe 4 weist einen hin und her verfahrbaren Stempel 13 antreibenden Servo-Motor 12 auf. Mit seiner Hilfe ist in einem Kolben 14 der Pumpe 4 ein Unterdruck erzeugbar, der in den Vakuumverband 1 geleitet wird. Der Unterdruck des Vakuumverbandes 1 ist der zwischen der Außenfläche der Wunde und den der Wunde zugewandten Innenlagen des Vakuumverbandes 1 messbare Druck. Zwar wird der Vakuumverband 1 nicht dauerhaft luftdicht auf der Haut des Patienten aufklebbar sein, jedoch ist es für eine erfindungsgemäße Wechseldruckbehandlung der Wunde hinreichend, einen periodisch impulsartigen Unterdruck im Vakuumverband 1 erzeugen zu können.

Zusätzlich ist über eine weitere elektrische Leitung 9 ein mit einer elektronischen Steuerung 11 in Verbindung stehender Pulsmesser 8 an einer Manschette 10 vorgesehen. Die Manschette 10 ist zur Pulsmessung um eine Extremität des Patienten schnallbar. Der Pulsmesser 8 misst einen Puls 21 des Patienten in einem von der Wunde beabstandeten Messbereich, z. B. am Oberarm, bei einer Unterarmwunde oder am Oberschenkel bei einer Unterschenkelwunde.

In Fig. 2 ist der im Messbereich gemessene Puls 21 das oberste Signal. Der gemessene Puls 21 weist gleichmäßig zeitlich voneinander beabstandete Pulsschläge 22 auf. Die Pulsschläge 22 sind mit dem Pulsmesser 8 messbar und in ihm in elektrische Pulsschlagsignale umwandelbar. Die elektrischen Pulsschlagsignale werden über die elektrische Verbindung 9 an die elektronische Steuerung 11 übermittelt. Die elektronische Steuerung 11 wertet die elektrischen Pulsschlagsignale aus und berechnet aus ihnen ein periodisch auftretende Steuerimpulse 24 aufweisendes Steuersignal 23. Das Steuersignal 23 ist unterhalb des Pulses 21 in Fig. 2 dargestellt. Das Steuersignal 23 ist phasengleich zum Puls 21 vorgesehen. Es können aber in anderen Ausführungsformen der Erfindung Phasenverschiebungen vorgesehen sein, die verschieden von Null sind, insbesondere bei unter 10° liegen.

Die Steuerimpulse 24 weisen jeweils eine zeitlich vorlaufende steile positive Flanke 24 a und eine zeitlich nachlaufende flache negative Flanke 24 b auf. Das Steuersignal 23 wird über das Steuerkabel 5 an den Servo-Motor 12 der Pumpe 4 geführt. Der Steuerimpuls 23 steuert den Servo-Motor 12 in der Weise, dass der Stempel 13 in Fig. 1 zunächst gemäß dem steilen Flankenverlauf 24 impulsartig nach innen in den Kolben 14 verfahren wird und so im Kolben 14 impulsartig einen Unterdruck erzeugt, der zeitlich etwas verzögert einen Unterdruckimpuls 26 im Vakuumverband 1 erzeugt. Der zeitliche Verlauf der Veränderung des Unterdrucks wird vom Druckmesser 6 im Vakuumverband gemessen. Die in ein elektrisches Unterdrucksignal 25 umgewandelten Wechseldruckmesswerte sind in der dritten Signalzeile der Fig. 2 dargestellt. Der Stempel 13 wird entlang einer flacheren negativen Flanke 24 b wieder langsamer, aber immer noch impulsartig zurückverfahren, so dass sich der Unterdruck 25 im Vakuumverband 1 impulsartig abbaut. Gleichzeitig kann ein einen Druckausgleich mit der Umgebungsluft ermöglichendes (nicht eingezeichnetes) Ventil im Luftschlauch 3 oder Kolben 14 geöffnet werden. Das im Vergleich zum Unterdruckaufbau langsamere Zurückverfahren des Stempels 13 trägt auch der Tatsache Rechnung, dass etwas Luft von außen in den Vakuumverband 1 eindringt und wieder hinaus gedrückt werden muss, ohne einen Überdruck im Vakuumverband 1 zu bewirken.

Statt eines Servo-Motors kann auch ein auf eine Membranpumpe wirkender Gleichstromhubmagnet vorgesehen sein. Die Gleichstromhubmagnet ist besonders kostengünstig und gestattet ein schnelle kurz ansprechende Steuerung der Membranpumpe.

Das Unterdrucksignal 25 weist gegenüber dem Steuersignal 23 eine Phasenverschiebung auf, die auf der zeitlichen Verzögerung beruht, bis sich der im Kolben 14 ausbildende Unterdruck in den Vakuumverband 1 fortgepflanzt hat.

Das Unterdrucksignal 25 wird über die elektrische Leitung 7 der elektronischen Steuerung 7 zugeführt. In der elektronischen Steuerung 7 ist eine Laufzeittabelle abgelegt, die die Laufzeit enthält, die der Pulsschlag 22 vom Messbereich (dem Oberarm oder Oberschenkel) zum Wundbereich (am Unterarm oder Unterschenkel) benötigt. Die Laufzeit wird vorab empirisch für Probanden unter Feststellung der Größe, des Alters und anderer Parameter und der Festlegung der Pulsmessstellen sowie den hypothetischen Wundbereichen, die durch eine entsprechend platzierte weitere Pulmessstelle bei einem gesunden Probanden simuliert werden, ermittelt. In einer vereinfachten Ausführungsform wird die Laufzeit vernachlässigt, da sie sich im Millisekundenbereich bewegt.

Dabei ist der Begriff der Laufzeit weit auszulegen. Es ist somit auch denkbar, dass der Puls an einem Arm des Patienten gemessen wird und der Wundbereich am anderen Arm des Patienten vorhanden ist. Die Laufzeittabelle speichert zeitliche Differenzen des Auftretens des Pulsschlags in verschiedenen Bereichen des menschlichen Körpers. Die Laufzeittabelle geht in die Berechnung des Steuersignals 23 ein.

Auf diese Weise stellt das medizinische Unterdruckgerät sicher, dass der Unterdruckimpuls 25 im Vakuumverband 1 zusammen mit dem Pulsschlag 28 des Pulses im Wundbereich 27 auftritt. Durch das Zusammenfallen von Unterdruckimpuls 26 und Pulsschlag 28 im Wundbereiche werden die Durchblutung und damit auch der Heilungsprozess, insbesondere der Wundränder, gefördert, während in den Ruhephasen zwischen den Pulsschlägen 28 der Abtransport des Wundsekretes gefördert wird.

Die Dauer des Unterdruckimpulses kann verstellt werden und auf den Patienten und die Art der Wunde angepasst werden. Ebenso ist denkbar, den Unterdruckimpuls genau während oder zeitlich vor nach dem Pulsschlag in Bereich der Wunde auftreten zu lassen.

Ebenso ist der Flankenverlauf des Unterdruckimpulses variabel einstellbar. Die vor- und nachlaufenden Flanken können symmetrisch zueinander ausgebildet sein. Es ist aber auch denkbar, dass der Druck nach dem Maximalwert des Unterdruckimpulses langsamer und flacher wieder ansteigt als vorher der Druck zum Maximalwert abgefallen ist. Auch das Gegenteil ist denkbar.

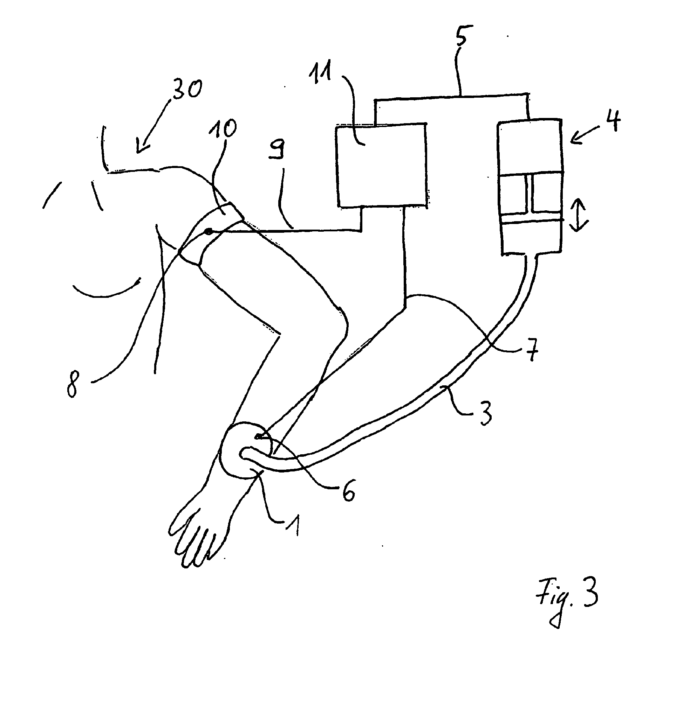

Fig. 3 zeigt das medizinische Unterdruckgerät schematisch in einer Anwendung. Die Manschette 10 mit Pulsmesser 8 ist um einen Oberarm des Patienten 30 geschnallt. Der gemessene Puls 21 wird der elektronischen Steuerung 11 zugeführt. Der Vakuumverband 1 ist um eine am Unterarm aufgetretene Wunde gelegt. Der Vakuumverband 1 weist den Drucksensor 6 auf, der das Unterdrucksignal 25 an die elektronische Steuerung 11 leitet. Die elektronische Steuerung 11 berechnet aus dem gemessenen Puls 21 und dem Unterdrucksignal 25 unter Berücksichtigung der in der Laufzeittabelle abgelegten Laufzeit zwischen gemessenem Puls 21 und Puls im Wundbereich 27 das Steuersignal 23.

## Patentansprüche

1. Medizinisches Gerät zur Verbesserung eines Wundheitungsprozesses eines Patienten (30) mit einer Absaugeinrichtung für Wundexsudate einer Wünde
mit einem Saugaufsatz (1) für die Wunde, der im aufgelegten Zustand einen im Wesentlichen luftdichten Abschluss (2) mit einem Hauptbereich des Patienten (30) erzeugt und
mit einer über eine druckleitende Verbindung (3) mit dem Saugaufsatz (1) in Verbindung stehenden steuerbaren Pumpe (4), die ein vom Luftdruck unabhängiges auf die Wunde wirkendes Wechseldrucksignal (25) mit periodisch auftretenden Wechseldruckimpulsen (26) im Saugaufsatz (1) erzeugt und
mit einem Pulsschläge (22) eines gemessenen Pulses (21) des Patienten (30) messenden Pulsmesser (8) und
mit einer mit dem Pulsmesser (8) und der Pumpe (4) in Verbindung stehenden Steuereinrichtung (11), die Pulsschläge (22) des gemessenen Pulses (21) auswertet und ein die Pumpe (4) steuerndes Steuersignal (23) in der Weise erzeugt, dass Pulsschläge (28) eines Pulses im Wundbereich (27) mit den Wechseldruckimpulsen (26) des Wechseldrucksignals (25) synchronisiert sind, **dadurch gekennzeichnet, dass**
die Steuereinrichtung (11) zur Erzeugung der Steuersignale (23) der Pumpe (4) von einer Absaugeinrichtung für Wundexsudate der Wunde entkoppelt ist.

2. Medizinisches Gerät nach Anspruch 1,
**gekennzeichnet durch** einen Speicher mit einer abgelegten Laufzeittabelle mit Zeitdifferenzen zwischen dem zeitlichen Auftreten der Pulsschläge (22) des gemessenen Pulses (21) und dem zeitlichen Auftreten der zugeordneten Pulsschläge (28) des Pulses im Wundbereich (27).

3. Medizinisches Gerät nach Anspruch 1 oder 2,
**gekennzeichnet durch** einen am Saugaufsatz (1) angeordneten Drucksensor (6) mit dem das Wechseldrucksignal (25) messbar ist, das an die Steuereinrichtung (11) abgebbar ist und **dadurch**, dass die Steuereinrichtung (11) das Wechseidrucksignal (25) mit dem Puls im Wundbereich (27) vergleicht und das Steuersignal (23) anpasst.

4. Medizinisches Gerät nach einem der vorstehenden Ansprüche,
**dadurch gekennzeichnet, dass** das Steuersignal (23) periodisch voneinander beabstandete Steuerimpulse (24) aufweist, deren periodischer Abstand im Wesentlichen einem periodischen Abstand der gemessenen Pulsschläge (22) entspricht.

5. Medizinisches Gerät nach einem der vorstehenden Ansprüche,
**dadurch gekennzeichnet, dass** die Steuereinrichtung (11) ein periodisches Steuersignal (23) mit Steuerimpulsen (24) erzeugt, die jeweils eine positive und eine negative Flanke (24a, 24b) aufweisen.

6. Medizinisches Gerät nach Anspruch 5,
**dadurch gekennzeichnet, dass** die Steuereinrichtung (11) eine Eingabeeinrichtung aufweist, mit der die Steigung und/oder Gestalt der Flanken (24a, 24b) der Steuerimpulse (24) einstellbar ist und/oder die Impulshöhe und/oder Tiefe der Steuerimpulse (24) einstellbar ist.

## Claims

1. A medical device for improving a wound healing process of a patient (30), comprising a suction unit for wound exudate of a wound,
comprising a suction pad (1) for the wound, which in the applied condition generates a substantially airtight closure (2) with a skin section of the patient (30), and
comprising a controllable pump (4) in communication with said suction pad (1) via a pressure-conducting connection (3), said pump generating an alternating pressure signal (25) that is independent of the air pressure and acts on the wound, said signal having periodically occurring alternating pressure pulses (26) in the suction pad (1), and
comprising a heart rate monitor (8) for measuring pulse beats (22) of a measured pulse (21) of the patient (30), and
comprising a control unit (11) in communication with said heart rate monitor (8) and said pump (4), which control unit evaluates the pulse beats (22) of the measured pulse (21) and generates a control signal (23) controlling said pump (4) in such a way that pulse beats (28) of a pulse in the wound region (27) are synchronised with the alternating pressure pulse (26) of said alternating pressure signal (25),
**characterised in that** the control unit (11) for generating the control signals (23) of the pump (4) is decoupled from a suction unit for wound exudate of the wound.

2. The medical device according to claim 1, **characterised by** a memory with a stored run-time table with time differences between the time of the occurrence of the pulse beats (22) of the measured pulses (21) and the time of the occurrence of the associated pulse beats (28) of the pulse in the wound region (27).

3. The medical device according to claim 1 or 2, **characterised by** a pressure sensor (6) provided on said suction pad (1), by means of which said alternating pressure signal (25) can be measured, which alternating pressure signal can be output to the control unit (11), and in that the control unit (11) compares said alternating pressure signal (25) with the pulse in the wound region (27) and adapts said control signal (23).

4. The medical device according to any one of the preceding claims, **characterised in that** the control signal (23) has control pulses (24) that are spaced apart by periodic intervals, the periodic distances between which substantially correspond to a periodic distance between the measured pulse beats (22).

5. The medical device according to any one of the preceding claims, **characterised in that** the control unit (11) generates a periodic control signal (23) with control pulses (24), which each have a positive and a negative edge (24a, 24b).

6. The medical device according to claim 5, **characterised in that** the control unit (11) has an input unit, by means of which the slope and/or the shape of said edges (24a, 24b) of said control pulses (24) is/are adjustable and/or the pulse height and/or depth of said control pulses (24) can be adjusted.

## Revendications

1. Appareil médical pour améliorer un processus de guérison de plaie d'un patient avec un dispositif d'aspiration pour les exsudats d'une plaie,
avec un embout d'aspiration (1) pour la plaie qui produit, à l'état appliqué, une adhérence substantiellement étanche à l'air (2) avec une zone de la peau du patient (30) et
avec une pompe pouvant être commandée (4) qui est en relation par une liaison conductrice de pression (3) avec l'embout d'aspiration (1), pompe qui produit un signal de pression alternative (25) qui agit sur la plaie, indépendant de la pression de l'air, avec des impulsions de pression alternative (26) qui apparaissent de manière périodique dans l'embout d'aspiration (1) et
avec un pulsomètre (8) qui mesure des battements (22) d'un pouls mesuré (21) du patient (30) et
avec un dispositif de commande (11), qui est en relation avec le pulsomètre (8) et la pompe (4), qui exploite les battements (22) du pouls mesuré (21) et qui produit un signal de commande (23) qui commande la pompe (4) de manière à ce que des battements (28) d'un pouls dans la zone de la plaie (27) soient synchronisés avec les impulsions de pression alternative (26) du signal de pression alternative (25),
**caractérisé en ce que** le dispositif de commande (11) est découplé d'un dispositif d'aspiration pour les exsudats de la plaie pour produire les signaux de commande (23) de la pompe (4).

2. Appareil médical selon la revendication 1,
**caractérisé par** une mémoire avec un tableau de durée de fonctionnement qui y est déposé avec des différences de temps entre l'apparition dans le temps des battements de pouls (22) du pouls mesuré (21) et l'apparition dans le temps des battements correspondants (28) du pouls dans la zone de la plaie (27).

3. Appareil médical selon la revendication 1 ou 2,
**caractérisé par** un capteur de pression (6) placé sur l'embout d'aspiration (1) avec lequel le signal de pression alternative (25) peut être mesuré, signal qui peut être donné au dispositif de commande (11), et par le fait que le dispositif de commande (11) compare le signal de pression alternative (25) avec le pouls dans la zone de la plaie (27) et adapte le signal de commande (23).

4. Appareil médical selon l'une des revendications précédentes,
**caractérisé en ce que** le signal de commande (23) présente des impulsions de commande (24) espacées périodiquement l'une de l'autre dont l'espacement périodique correspond sensiblement à un espacement périodique des battements de pouls mesurés (22).

5. Appareil médical selon l'une des revendications précédentes,
**caractérisé en ce que** le dispositif de commande (11) produit un signal de commande périodique (23) avec des impulsions de commande (24) qui présentent respectivement un flanc positif et un flanc négatif (24a, 24b).

6. Appareil médical selon la revendication 5,
**caractérisé en ce que** le dispositif de commande (11) présente un dispositif d'entrée avec lequel la pente et/ou la configuration des flancs (24a, 24b) des impulsions de commande (24) est réglable et/ou la hauteur des impulsions et/ou la profondeur des impulsions de commande (24) est réglable.
